# EUROPEAN PATENT APPLICATION

(11) **EP 3 756 646 A1**
(43) Date of publication of application: **30.12.2020**
(21) Application number: 19753939.8
(22) Date of filing: 18.02.2019
(51) Int. Cl.: A61K 8/81, A61K 8/04, A61Q 19/00, B01J 13/00

(54) **DECORATIVE AQUEOUS COMPOSITION AND METHOD FOR PRODUCING SAME**

(30) Priority: 19.02.2018 JP 2018026682
(71) Applicant: Public University Corporation Nagoya City University, Mizuho-ku Nagoya-shi Aichi 467-8601 (JP); Daicel Corporation, Osaka 530-0011 (JP)
(72) Inventor: YAMANAKA Junpei, Nagoya-shi, Aichi 467-8603 (JP); TOYOTAMA Akiko, Nagoya-shi, Aichi 467-8603 (JP); OKUZONO Tohru, Nagoya-shi, Aichi 467-8603 (JP); YAMAMOTO Ruri, Nagoya-shi, Aichi 467-8603 (JP); FUKUSHIMA Ayanori, Nagoya-shi, Aichi 467-8603 (JP); SATOU Yui, Nagoya-shi, Aichi 467-8603 (JP); SAKANISHI Yuichi, Tokyo 108-8230 (JP); FUKUDA Hitomi, Tokyo 108-8230 (JP)
(74) Representative: Grünecker Patent- und Rechtsanwälte PartG mbB
(86) International application number: PCT/JP2019/005758
(87) International publication number: WO 2019/160132

(57) **Abstract**

[Object]

To provide a decorative aqueous composition that includes particles formed from opal- type colloidal crystals uniformly and stably dispersed in an aqueous dispersion medium, and exhibits a structural color due to interference of light, and to provide a method for producing the decorative aqueous composition.

[Solution]

The decorative aqueous composition of the present invention contains particles of opal- type colloidal crystals dispersed in an aqueous dispersion medium in which a polymer is dissolved. The particles of the opal- type colloidal crystals are made from hydrophilic colloidal particles having an average particle diameter ranging from 10 nm to 1000 nm with a variation coefficient of the particle diameter being within 20%. Therefore, the decorative aqueous composition of the present invention contains opal- type colloidal crystals uniformly and stably dispersed in an aqueous dispersion medium, and exhibits a structural color due to interference of light. As a result, the decorative aqueous composition can be suitably used in a cosmetic lotion or the like.

## Description

### Technical Field

The present invention relates to a decorative aqueous composition that contains opal-type colloidal crystals dispersed in an aqueous dispersion medium and exhibits a structural color through Bragg diffraction, and to a method for producing the same.

### Background Art

The term "colloidal crystal" refers to a substance in which particles having a uniform particle diameter with a size ranging from several nm to several µm form an ordered structure that is periodically and regularly aligned. Similar to normal crystals, colloidal crystals cause Bragg diffraction of electromagnetic waves according to the lattice spacing. The diffraction wavelength can be set to the visible light region, the infrared region, or other various wavelength regions by selecting production conditions (such as the particle concentration, particle diameter, and refractive index of the particles or medium). Therefore, technology has been developed in which colloidal crystals are dispersed in an aqueous dispersion medium to form a glittering decorative aqueous composition (for example, Patent Documents 1 to 3).

Three types of colloid crystals are well known.

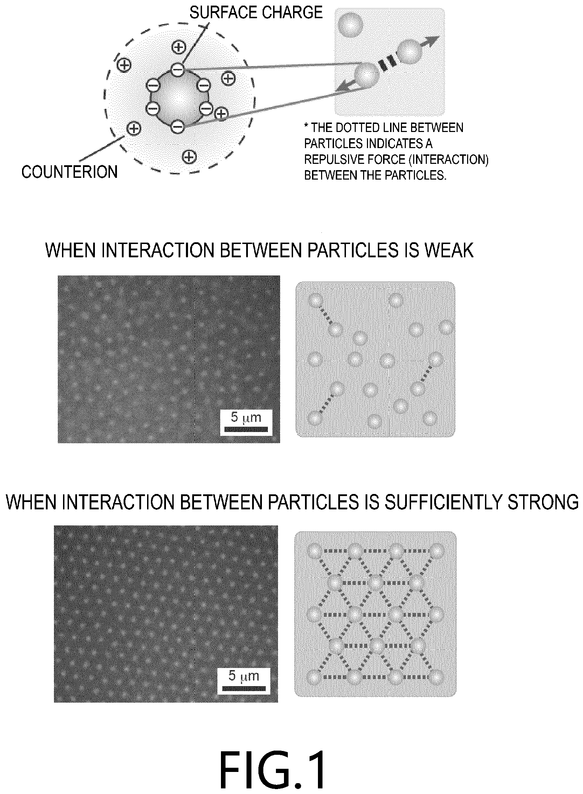

The first type is a charged colloidal crystal. This is formed in charged colloidal system in which colloidal particles charged by a surface charge dispersed with electrostatic repulsion exerted between the particles. As illustrated in FIG. 1, when the electrostatic repulsion is small, the colloidal particles move around freely through Brownian motion, and are therefore randomly positioned. However, as the electrostatic repulsion increases, the particles tend to separate as far as possible from the other particles, and as a result, the particles form a colloidal crystal with the particles aligned at a certain lattice spacing. Since the electrostatic repulsion reaches a long distance, crystals are formed at a low particle concentration (that is, where the distance between particles is long).

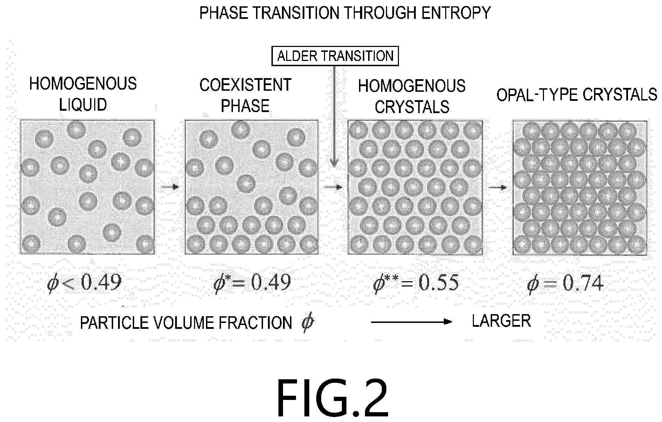

The second type is a hard sphere-type colloidal crystal in which only hard sphere repulsion is exerted between the particles. When a large number of macroscopic spheres are packed in a limited space, the spheres align in a periodic crystal-like manner, and the hard sphere-type colloidal crystals resemble this phenomenon. The parameter governing crystallization is only the particle volume fraction φ in the dispersion, and as illustrated in FIG. 2, crystallization occurs around φ = 0.49 (referred to as the Alder transition). At high concentration conditions of φ > 0.49, the number of possible particle arrangements is greater for a periodic particle arrangement than for a random particle arrangement, and therefore entropy is larger in a crystalline state than in a disordered state. Accordingly, in the hard sphere-type colloidal crystal, a crystal structure is formed in a state where the particles are not in contact with each other, even if in a close-packed condition.

The third type is an opal-type colloidal crystal, and as illustrated in the figure at the furthest right side of FIG. 2, the opal-type colloidal crystal has a crystalline structure packed with mutually contacting particles. Opals that are used as jewels are formed as colloidal crystals in which silica (SiO₂) microparticles having a uniform particle diameter are precipitated and close-packed, and colloidal crystals in which colloidal microparticles are close-packed and crystallized are commonly referred to as opal-type colloidal crystals. The volume fraction of such crystals varies depending on the crystal structure, but for example, the volume fraction is approximately 0.68 with a body-centered cubic lattice and approximately 0.74 with a face-centered cubic lattice.

Of the three types of colloidal crystals described above, the decorative aqueous compositions described in the abovementioned Patent Documents 1 to 3 use charged colloidal crystals. That is, the decorative aqueous compositions thereof have charged colloidal crystals that are formed in an aqueous dispersion medium, and result in liquids having a glittering structural color due to the interference of visible light. Therefore, application in cosmetic lotions and the like has been proposed.

However, as the salt concentration of charged colloidal system increases, electrical repulsion is reduced, which makes it difficult to keep the colloidal particles at a constant distance. For example, when the colloidal particles are diluted, charged colloidal crystals do not form at salt concentrations of several 10 µM or greater. In addition, when the concentration of colloidal particles is greater than or equal to 10%, charged colloidal crystals do not form at a salt concentration of approximately 0.1 mM or higher. Therefore, stable charged colloids cannot be formed at a salt concentration of 0.1 mM or higher. Cosmetic products such as cosmetic lotions contain ionic additives and pH buffers, and therefore use of charged colloidal crystals in decorative applications of cosmetic products or such is difficult from a practical perspective.

On the other hand, hard sphere-type colloidal crystals require a high concentration of particles with the volume fraction of the particles being 0.5 or greater, and therefore it is difficult to use such hard sphere-type colloidal crystals in decorative applications of cosmetic products or the like. In addition, opal-type colloidal crystals require an even higher concentration of particles with the volume fraction of the particles being 0.74 or greater, and therefore it becomes even more difficult to use opal-type colloidal crystals in decorative applications of cosmetic products or the like.

It is also conceivable to pulverize the hard sphere-type colloidal crystals and the opal-type colloidal crystals and disperse them in a dispersion medium to exhibit structural color, but it is difficult to stably disperse the finely pulverized matter of these colloidal crystals in a liquid for a long period of time, and uniform color development is of course difficult.

Note that the inventors of the present invention discovered that when a method of dissolving a polymer in a colloidal system in which two types of colloidal particles having a uniform particle diameter are dispersed is used to produce opal-type colloidal crystals, the precipitated colloidal particles become an opal-type colloidal eutectic substance (Patent Document 4). In this method, colloidal particles precipitate to form a eutectic structure, and the opal-type colloidal crystals are not dispersed in the dispersion medium. And thus, a decorative aqueous composition in which the opal-type colloidal crystals are dispersed in an aqueous solvent is not formed.

In addition, though an aqueous dispersion medium is not used, a phenomenon was discovered in which, using ethyl naphthalene as the dispersion medium, in a mixed system of two types of crosslinked polystyrene particles and one type of linear polystyrene polymer with the mixed system a plurality of types of opal-type colloidal crystals having different lattice constants are dispersed and coexist in the ethyl naphthalene (Non-Patent Document 1).

### Citation List

### Patent Documents

Patent Document 1: JP 06-100432 A
Patent Document 2: JP 3537156 B
Patent Document 3: JP 05-320022 A
Patent Document 4: WO 2016/093121

### Non-Patent Document

Non-Patent Document 1: Anna Kozina, et al., Soft Matter, 10, 9523-9533 (2014)

### Summary of Invention

### Technical Problem

The present invention is completed in light of the foregoing circumstances, and an object of the present invention is to provide a decorative aqueous composition that contains opal-type colloidal crystals uniformly and stably dispersed in an aqueous dispersion medium, and exhibits a structural color due to the interference of light, and to provide a method for producing the same.

### Solution to Problem

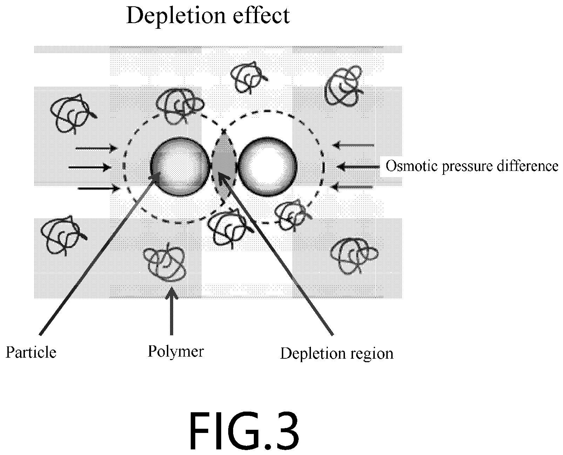

In a case where a polymer that is soluble in a dispersion medium is added in a colloidal dispersion, when the colloidal particles are in close proximity to each other, as illustrated in FIG. 3, a region where the space is too narrow for the dissolved polymer to enter is formed (hereinafter, referred to as a "depletion region"). Therefore, there is a difference in osmotic pressure due to a difference in concentrations of the polymers between the depletion region and other regions. It is known that a close-packed opal-type colloidal crystal is formed, in which the colloidal particles are in close proximity to, and are in contact with each other. Using this phenomenon, the present inventors discovered that by dissolving the polymer in a colloidal system in which colloidal particles are dispersed in an aqueous dispersion medium, a decorative aqueous composition having opal-type colloidal crystals dispersed in an aqueous dispersion medium is formed, and the present inventors thereby completed the present invention.

Namely, a decorative aqueous composition of the present invention has opal-type colloidal crystals dispersed in an aqueous dispersion medium, in which a polymer is dissolved, and colloidal particles constituting the opal-type colloidal crystals are dispersible in the aqueous dispersion medium and have an average particle diameter ranging from 10 nm to 1000 nm, and a variation coefficient of the particle diameter is within 20%.

With the decorative aqueous composition of the present invention, a polymer is dissolved in an aqueous dispersion medium (in the present specification, the "aqueous dispersion medium" is defined as a dispersion medium containing 50 wt.% or more of water), and therefore a depletion region in which the polymer cannot enter is formed in a vicinity where colloidal particles constituting the opal-type colloidal crystals are in close proximity of each other. As a result, there is a difference in osmotic pressure the depletion region and other regions, and the colloidal particles are brought into contact with each other to form opal-type colloidal crystals. This difference in osmotic pressure is mostly stable regardless of the concentration of the electrolyte, and therefore even if the electrolyte concentration is at 0.1 mM, the crystal structure does not collapse. Note that the dissolved polymer (hereinafter, also referred to as a "depletant") need not necessarily be a linear polymer, and may also be a branched polymer, or microparticles of a size differing from the microparticles constituting the crystals. In the examples, representative examples are given using linear polymers, but the present invention is not limited to linear polymers.

In addition, the variation coefficient of the particle diameter of the colloidal particles constituting the opal-type colloidal crystals is within 20%, and therefore it is possible to achieve an orderly colloidal crystal structure with few defects. Here, the variation coefficient of the particle diameter refers to the standard deviation of the particle diameter × 100/average particle diameter.

Furthermore, since the average particle diameter of the colloidal particles ranges from 10 nm to 1000 nm, electromagnetic waves of various wavelengths such as ultraviolet rays, visible light rays, and infrared rays can be diffracted. Therefore, a composition having decorative properties can be formed.

Accordingly, the decorative aqueous composition of the present invention contains opal-type colloidal crystals uniformly and stably dispersed in an aqueous dispersion medium, and exhibits a structural color due to the interference of light.

The specific gravity of the colloidal particles constituting the opal-type colloidal crystal particles is preferably in a range from 0.9 times to 1.1 times the specific gravity of the aqueous dispersion medium. When the specific gravity of the colloidal particles is in this range, the specific gravity of the colloidal particles is close to the specific gravity of the aqueous dispersion medium, and therefore the opal-type colloidal crystal particles do not easily precipitate and can be stably dispersed. To bring the specific gravity of the aqueous dispersion medium close to the specific gravity of the colloidal particles constituting the opal-type colloidal crystals, a solute such as sugar may be added to the aqueous dispersion medium. On the other hand, even when the density of the particles is large, decoration using sedimentation is conceivable.

Furthermore, the polymer dissolved in the aqueous dispersion medium of the decorative aqueous composition according to an embodiment of the present invention is not limited, and in addition to a vinyl-based polymer (such as an acrylic acid-based polymer and a methacrylic acid-based polymer), a water soluble polysaccharide (such as carboxymethyl cellulose, hyaluronic acid, or alginic acid) can be suitably used. From the perspective of facilitating formation of a depletion region between colloidal particles, the average molecular weight (here, "average molecular weight" refers to the number average molecular weight, same hereinafter) is preferably 10000 or higher, and the average molecular weight is more preferably 100000 or higher.

The colloidal particles that form an opal-type colloidal crystal dispersed in a dispersion medium of the decorative aqueous composition according to an embodiment of the present invention are colloidal particles formed from a crosslinked polymer. If the colloidal particles are formed from such polymers, the dispersion medium can freely pass through the crosslinked mesh structure, and thus the apparent specific gravity of the colloidal particles becomes very close to that of the dispersion medium. Therefore, the apparent specific gravity of an opal-type colloidal crystal including colloidal particles is extremely close to that of the dispersion medium, and the opal-type colloidal crystals are stably dispersed in the dispersion medium without precipitating. In addition, since the refractive index of the solvent is also close to that of the colloidal particles, the transparency of the dispersion is enhanced.

Moreover, the crosslinked polymer is not limited, and examples thereof include polymers having constituent units of acrylamide, water soluble acrylamide derivatives (such as methylolacrylamide and dimethyl acrylamide), acrylic acid, and water soluble acrylic acid derivatives (such as hydroxyethyl acrylate). Furthermore, polysaccharides and derivatives thereof can also be used.

In addition, from the perspective of ensuring that the aqueous dispersion medium easily passes through the crosslinked mesh structure, the crosslinked polymer preferably has good hydrophilicity.

The decorative aqueous composition according to an embodiment of the present invention exhibits a beautiful glittering structural color due to the interference of visible light by the dispersed opal-type colloidal crystals, and therefore, the decorative aqueous composition can be used in cosmetic products such as cosmetic lotions or milky lotions.

Furthermore, the decorative aqueous composition according to an embodiment of the present invention can be used as a sensing material for temperature, pH, or the like. When colloidal particles that changes the particle diameter depending on the temperature or pH of the medium are used, the diffraction wavelength of the colloidal microcrystals formed by these particles changes depending on the temperature or pH. Therefore, such changes can be used for visual or spectral sensing.

The decorative aqueous composition according to an embodiment of the present invention can also be a decorative solidified body that has been solidified using a solidifying agent. As a result, colloidal crystal particles are immobilized, and the mechanical strength dramatically increases. As a result, the material is extremely easy to handle. In addition, the lattice spacing of the colloidal crystals changes when pressure is applied to the solidified body, and therefore the solidified body can be used as a pressure sensor or the like by measuring the change in the reflection spectrum thereof.

The decorative aqueous composition according to an embodiment of the present invention can be produced by mixing an aqueous dispersion medium, a polymer soluble in the aqueous dispersion medium, and colloidal particles having an average particle diameter ranging from 10 nm to 1000 nm with a variation coefficient of the particle diameter being within 20%, to form a dispersion of particles including opal-type colloidal crystals.

### Brief Description of Drawings

FIG. 1 is a schematic diagram illustrating the formation of a charged colloidal crystal.
FIG. 2 is a schematic diagram illustrating a phase transition of a colloidal system.
FIG. 3 is a schematic diagram illustrating a depletion effect due to dissolution of a polymer in a colloidal system.
FIG. 4 is a schematic diagram of a decorative aqueous composition according to an embodiment of the present invention.
FIG. 5 is a schematic view of a colloidal particle made from a crosslinked polymer.
FIG. 6 is a process diagram illustrating a method for producing a decorative aqueous composition.
FIG. 7 is photographs of a decorative aqueous composition prepared in Example 1.
FIG. 8 is a graph showing measurement results for the decorative aqueous composition prepared in Example 1, the results being obtained by measuring the reflection spectrum at a different height from the bottom of a measurement cell.
FIG. 9 is a graph showing measurement results for a decorative aqueous composition prepared in Comparative Example 2, the results being obtained by measuring the reflection spectrum at a different height from the bottom of the measurement cell.
FIG. 10 is photographs showing the dispersed state when microgels prepared in Synthesis Examples 1 to 4 were dispersed in water-ethanol solutions at various temperatures.
FIG. 11 is a graph showing the measurement results of the reflection spectra of microgels prepared in Synthesis Example 4.
FIG. 12 is photographs of the decorative aqueous composition of Example 9.
FIG. 13 is photographs of the decorative aqueous compositions of Examples 11 to 13.
FIG. 14 is graphs showing the relationship between the concentration of the polymer dissolved in the decorative aqueous composition and the lattice spacings.

### Description of Embodiments

Hereinafter, embodiments that embody the present invention will be described with reference to the drawings.

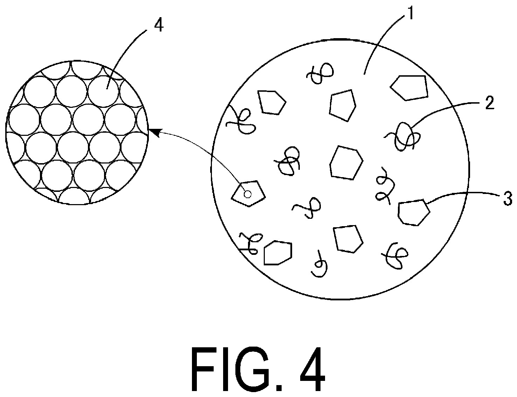

FIG. 4 is a schematic diagram of a decorative aqueous composition according to an embodiment of the present invention. In this decorative aqueous composition, a polymer 2 having an average molecular weight of 100000 or higher is dissolved in an aqueous dispersion medium 1 such as water or water-ethanol (50% or more of water in terms of a weight ratio), and opal-type colloidal crystals 3 are further dispersed in the aqueous dispersion medium 1. Each opal-type colloidal crystal 3 has a structure in which colloidal particles 4 are close-packed. The average particle diameter of the colloidal particle 4 ranges from 10 nm to 1000 nm, and a variation coefficient of the particle diameter is within 20%.

In this decorative aqueous composition, the polymer 2 having an average molecular weight of 100000 or higher is dissolved in the aqueous dispersion medium 1, and therefore as illustrated in FIG. 3, a depletion region of polymer (depletant) is formed in the vicinity where colloidal particles 4 constituting the opal-type colloidal crystal 3 are in close proximity to each other. As a result, a difference in osmotic pressure (osmotic pressure difference) is produced between the depletion region and other regions, which causes the colloidal particles 4 to contact with each other, and thus the opal-type colloidal crystal 3 is formed. The average molecular weight of the polymer is preferably 200000 or greater, more preferably 300000 or greater, and even more preferably 500000 or greater. The osmotic pressure difference is mostly stable regardless of the concentration of the electrolyte. Therefore, even if the electrolyte concentration of the solvent is at 0.1 mM, the structure of the opal-type colloidal crystal does not collapse.

In addition, the variation coefficient of the particle diameter of the colloidal particles 4 constituting the opal-type colloidal crystals 3 is within 20%, and therefore it is possible to achieve an orderly colloidal crystal structure with few defects. Furthermore, since the average particle diameter of the colloidal particles 4 ranges from 10 nm to 1000 nm, electromagnetic waves of various wavelengths such as ultraviolet rays, visible light rays, and infrared rays can be diffracted. Therefore, a composition having decorative properties can be formed.

Accordingly, light interference occurs due to the opal-type colloidal crystals 3, and as a result, the decorative aqueous composition according to an embodiment of the present invention exhibits glittering structural color.

The variation coefficient of the particle diameter of the colloidal particles constituting the opal-type colloidal crystals is required to be within 20%, is preferably less than 15%, more preferably less than 10%, and even more preferably less than 5%.

In addition, the specific gravity of the colloidal particles constituting the opal-type colloidal crystal particles is preferably in a range of from 0.9 times to 1.1 times the specific gravity of the aqueous dispersion medium. When the specific gravity of the colloidal particles is in this range, the specific gravity of the colloidal particles is close to the specific gravity of the aqueous dispersion medium, and therefore the opal-type colloidal crystal particles do not easily precipitate and can be stably dispersed. The specific gravity of the colloidal particles constituting the opal-type colloidal crystal particles more preferably ranges from 0.99 times to 1.01 times, and most preferably from 0.995 times to 1.005 times.

Furthermore, the polymer dissolved in the aqueous dispersion medium of the decorative aqueous composition can be used as long as it is a polymer that dissolves in the aqueous dispersion medium. The average molecular weight of the polymer is preferably 10000 or greater. As described above, when the colloidal particles are in close proximity to each other, the region where the space is too small for the polymer to enter (that is, the depletion region) is formed, and this region increases in its size. This increase of the depletion region in size brings the particles to be close together due to the osmotic pressure difference, and thereby ensuring the formation of the opal-type colloidal crystals. A polymer having an average molecular weight of 100000 or greater is even more preferable. Furthermore, as a preferable characteristic of the polymer, the polymer preferably has a property of not easily being adsorbed to the colloidal particles dispersed in the aqueous dispersion medium. This is because in a case where the polymer is easily adsorbed to the colloidal particles, the polymer may act as an agglomerating agent, and there is a risk that the colloidal particles may aggregate and precipitate.

In an embodiment of the present invention, a water-soluble ionic polymer or water-soluble nonionic polymer can be used as the water soluble polymer (depletant) that is added to express depletion attraction force between colloidal particles. Examples of water-soluble nonionic polymers include polyethylene glycol, polypropylene glycol, polyvinyl alcohol, polyether, and polyvinylpyrrolidone. Furthermore, examples of water-soluble ionic polymers include cationic polymers such as polyvinyl pyridine, polyvinyl benzyl ammonium, and polypeptides; and anionic polymers such as polyacrylic acids, polyacrylamides, poly(N-isopropylacrylamide), polypeptides and other such biopolymers, and natural polymers such as hyaluronic acid, chondroitin sulfate, alginic acid and other such polysaccharides. In the selection of these polymers, a polymer having a charge opposite to the surface charge of the colloidal particles is not preferable because such polymer may be adsorbed to the colloidal particles to form a charged colloid. However, even with a polymer having a charge opposite to the surface charge of the colloidal particles, if the salt concentration is high, the electric double layer becomes extremely thin, and the colloidal particles can be brought close to each other. As a result, a difference in osmotic pressure due to the difference in concentration of the polymer is created, which induces a depletion attraction force. Thus, the opal-type colloidal crystal is formed, and such polymer can be used. Furthermore, this phenomenon may be advantageously employed; the concentration of the salt in the colloid is adjusted as appropriate to control the thickness of the electric double layer, the attraction force between colloidal particles can be controlled. And thus, the growth rate of the opal-type colloidal crystals, the half width at half maximum of the colloidal crystal with respect to light, and the like can be controlled. Examples of methods for controlling the salt concentration include controlling the content rate of ionic groups (such as carboxylic acid salts and amino groups) in the polymer, and adding salt. In addition, the polymer is not limited to a linear polymer, and various branched polymers, polymer assemblies, micelles, and spherical particles can also be used as long as they exhibit a depletion effect and an attractive force effect.

Furthermore, the colloidal particles forming the opal-type colloidal crystals are preferably particles made from a hydrophilic polymer. Such colloidal particles can facilitate a stable dispersion of the opal-type colloidal crystal particles in the aqueous dispersion medium. Examples of the polymer constituting such colloid particles include poly(N-isopropylacrylamide), polyethylene glycol, polypropylene glycol, polyvinyl alcohol, polyether, and polyvinylpyrrolidone. Mixtures or copolymers of these polymers can also be used as the colloidal particles.

Furthermore, examples of water-soluble ionic polymers include cationic polymers such as polyvinyl pyridine, polyvinyl benzyl ammonium, and polypeptides; and anionic polymers such as polyacrylic acids, polyacrylamides, polypeptides, hyaluronic acid, chondroitin sulfate, alginic acid and other such polysaccharides, and other such natural polymers.

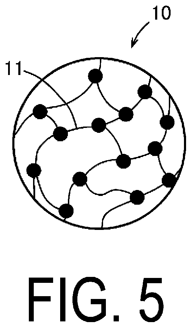

More preferable is a hydrophilic colloid particle 10 made from a crosslinked polymer, as illustrated in FIG. 5. The colloid particle 10 has a structure in which the polymer 11 is crosslinked with a crosslinking agent, and the dispersion medium can freely pass through the crosslinked mesh structure. Therefore, the apparent specific gravity of the colloidal particles 10 becomes extremely close to that of the dispersion medium, the apparent specific gravity of the opal-type colloidal crystals made from the colloidal particles 10 also becomes extremely close to that of the dispersion medium, and the opal-type colloidal crystals are stably dispersed in the dispersion medium without precipitating. The water-soluble polymer described above is desirable as the polymer constituting such a gel.

On the other hand, even hydrophobic particles such as styrene can be used when a dissociable group is introduced on the surface, because the hydrophobic particles can be stably dispersed in the dispersion medium. Examples of such polymers include polystyrene that has been made hydrophilic through copolymerization of styrene sulfonic acid.

The decorative aqueous composition according to an embodiment of the present invention can also be made into a decorative solidified body that has been solidified using a solidifying agent. As a result, mechanical strength can be dramatically increased, and handling ease is highly improved. In addition, the lattice spacing of the colloidal crystals changes when pressure is applied to the solidified body, and therefore the solidified body can be used as a pressure sensor or the like by measuring the change in the reflection spectrum thereof.

Examples of the solidifying agent include solutions containing a gel monomer, a crosslinking agent and a photopolymerization initiator. Examples of the gel monomer include vinyl monomers such as acrylamide and derivatives thereof, examples of the crosslinking agent include N,N'-methylene bisacrylamide, and examples of the photopolymerization initiator include 2,2'-azobis [2-methyl-N- [2-hydroxyethyl]-propionamide]. In addition, a water-soluble photosensitive resin in which an azide-based photosensitive group is pendant to polyvinyl alcohol can also be used. Furthermore, the colloid crystals may be formed in the resin monomer and then solidified.

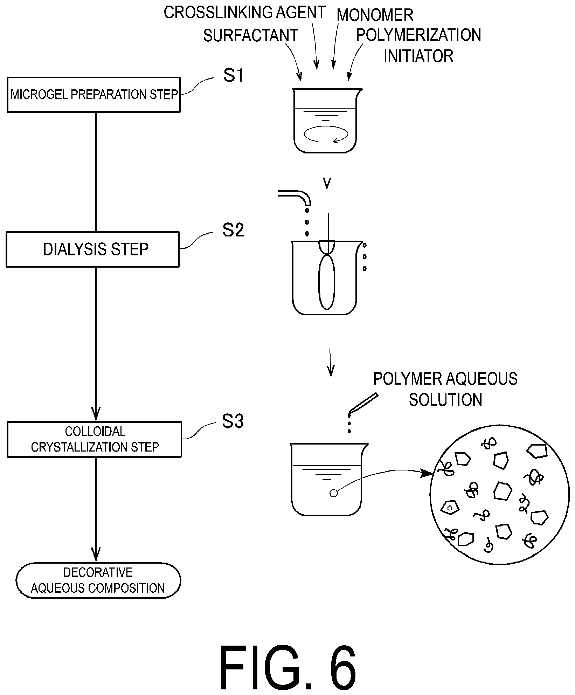

The decorative aqueous composition according to an embodiment of the present invention can be produced according to the processes illustrated in FIG. 6.

### Microgel Preparation S1

First, as a microgel preparation S1, hydrophilic colloidal particles 10 made from a crosslinked polymer are prepared as illustrated in FIG. 5. That is, a monomer that serves as a material of a polymer, a crosslinking agent, a surfactant, and a polymerization initiator are dispersed and mixed in purified water, and then heated to thereby carry out emulsion polymerization. This results in a dispersed liquid in which the microgels formed from the crosslinked polymer illustrated in FIG. 5 are dispersed in the dispersion medium.

### Dialysis S2

Furthermore, the microgel dispersion formed as described above is dialyzed against water, and the surfactant and polymerization initiator are removed (dialysis S2).

### Colloidal Crystallization S3

A polymer aqueous solution is added to the microgel dispersion purified by dialysis, and then stirred. This immediately results in a decorative aqueous composition in which opal-type colloidal crystals are dispersed, with the entire sample uniformly exhibiting a structural color.

### Examples

The present invention will be described below while comparing more specific examples according to an embodiment of the present invention with comparative examples.

### Example 1

### Preparation of a Microgel

N-isopropylacrylamide (NIPAM, Wako Pure Chemical Industries, Ltd.) was used as a monomer, N,N'-methylenebisacrylamide (BIS, Wako Pure Chemical Industries, Ltd.) was used as a crosslinking agent, sodium dodecyl sulfate (SDS, Wako Pure Chemical Industries, Ltd.) was used as a surfactant, and potassium peroxodisulfate (KPS, Kanto Chemical Co., Inc.) was used as a radical polymerization initiator. NIPAM (3.93 g) was dissolved in 175 mL of pure water produced in a Milli-Q purification device (hereinafter, referred to as "pure water"), BIS (0.075 g) was dissolved in 10 mL of pure water, and SDS (0.075 g) was dissolved in 60 mL of pure water. The three solutions were mixed and then transferred to a 500 mL 4-neck round bottom flask and stirred for 1 hour at 850 rpm and 70°C while circulating Ar. Separately, a solution in which KPS (0.15 g) was dissolved in 5 mL of pure water and held at 70°C was added to the 4-neck round bottom flask described above, and the mixture was stirred for an additional 4 hours at 850 rpm and 70°C while circulating Ar to carry out an emulsion polymerization reaction, and a dispersion of microgels, in which polyisopropylacrylamide (PNIPAM) was crosslinked by N,N'-methylenebisacrylamide (BIS), was obtained.

### Preparation of a Decorative Aqueous Composition

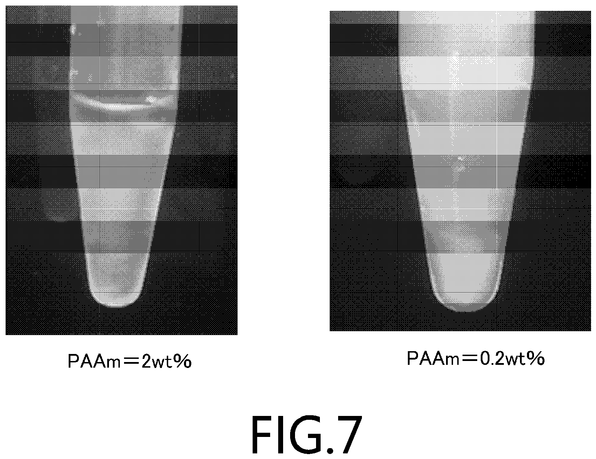

The microgel dispersion obtained as described above was purified by dialysis with pure water and diluted to approximately 2 wt.%. An aqueous solution of polyacrylamide (PAAm, average molecular weight of 400000) was added to the sample at an amount so as to become 2 wt.%, and the entire sample immediately exhibited a uniform structural color (FIG. 7, right). The entire sample immediately exhibited a uniform structural color even when the PAAm concentration was 0.2 wt.%, but with the passage of time, the material precipitated to the bottom of the container in which the sample was placed, and a structural color was observed in the precipitate (FIG. 7, left).

### Comparative Example 1

In Comparative Example 1, PAAm was not added to the microgel dispersion described above. All other details were the same as those of Example 1, and thus a detailed description thereof is omitted.

### Comparative Example 2

In Comparative Example 2, the microgels in the microgel dispersion of Comparative Example 1 were forcibly precipitated by centrifugation, and the microgel concentration was set to 40 wt.%.

### Evaluation

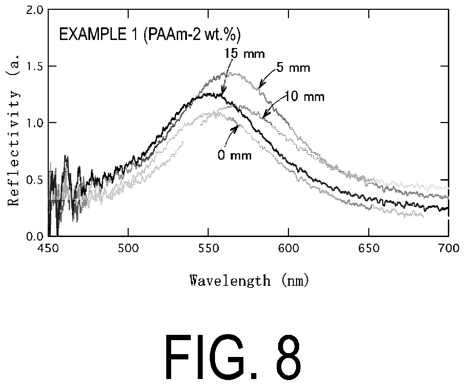

The reflection spectrum of the decorative aqueous composition of Example 1 was measured using a fiber spectrometer. The reflection spectrum of a sample with a PAAm concentration of 2 wt.% was measured at different heights (h) from the bottom of the measurement cell. As a result, as illustrated in FIG. 8, the reflection spectrum was approximately the same shape regardless of the height from the bottom, and the distance between the particle centers determined by the Bragg equation at the peak position was approximately 250 nm. This value indicates that the microgels were in mutual contact, and very small opal-type colloidal crystals were formed, and suggests crystallization by depletion attraction force (see FIG. 3).

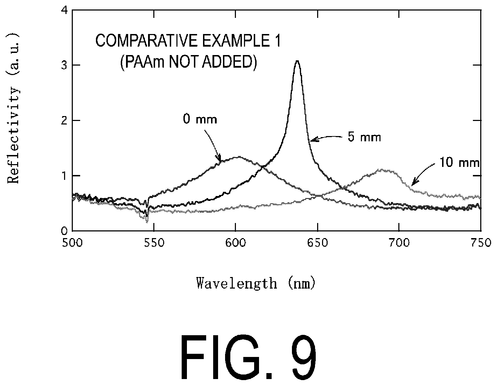

In contrast, in Comparative Example 1 in which the polymer (PAAm) was not added to the microgels, the entire sample did not exhibit a structural color. Therefore, in Comparative Example 2, the microgels were forcibly precipitated using a centrifugal separator and the microgel concentration was set to approximately 40 wt.% or higher, and a structural color was observed. The reflection spectrum of Comparative Example 2 is illustrated in FIG. 9. At this time, the structural color was expressed only in the lower portion of the centrifuge cell (area where the microgels were concentrated to a high concentration). In addition, the reflection peak wavelength varied depending on the height (h) from the bottom of the measurement cell, and the distance between particle centers determined by the Bragg equation varied in a range of from 290 nm to 310 nm. From the above results, it was found that in Comparative Example 2, hard sphere-type colloidal crystals in which only hard sphere repulsion acted on the microgel particles were formed, and the microgels were not in contact with each other.

### Example 2 and Example 3

In Example 2 and Example 3, sodium chloride (NaCI) was added to pure water serving as a dispersion medium, at an amount to arrive at a predetermined concentration (0.1 mM in Example 2 and 0.01 mM in Example 3). All other conditions were the same as those in Example 1 (polyacrylamide concentration of 2 wt.%), and thus a detailed description thereof is omitted.

### Example 4

In Example 4, hydrochloric acid (HCl) was added to pure water serving as a dispersion medium, at an amount to arrive at a concentration of 0.01 mM. All other conditions were the same as those in Example 1 (polyacrylamide concentration of 2 wt%), and thus a detailed description thereof is omitted.

### Example 5

In Example 5, sodium hydroxide (NaOH) was added to pure water serving as a dispersion medium, at an amount to arrive at a concentration of 0.01 mM. All other conditions were the same as those in Example 1 (polyacrylamide concentration of 2 wt%), and thus a detailed description thereof is omitted.

### Evaluation

With the decorative aqueous compositions of Example 2 to 5 prepared as described above, similar to the case of Example 1, the entirety of each sample immediately and uniformly exhibited the similar structural color as Example 1 after the addition of PAAm. In addition, the measurement results of the reflection spectra matched the case of Example 1 within the range of measurement error. From the foregoing, it was found that even in an aqueous solution with a salt concentration of 0.1 mM, the opal-type colloidal crystals were stably dispersed and exhibited a structural color. Therefore, the decorative aqueous compositions of Examples 1 to 5 can be sufficiently used in cosmetic lotions or the like that contains ionic additives or pH buffers.

### Example 6

In Example 6, the decorative aqueous composition of Example 1 was purified by dialysis, and then subjected to lyophilization to obtain a powder. When this powder was added to a 2 wt.% PAAm aqueous solution, it was found that a structural color was observed when the gel concentration was 2 wt.% or higher, and a dispersion of the opal-type colloidal crystals was obtained.

### Example 7

To 250 µL of a purified PNIPA gel (3.56 wt.%) aqueous solution prepared in the same manner as in Example 1, 25 mg of a polyacrylic acid (PAA, average molecular weight of 1000000) aqueous solution (prepared with a PAA concentration = 5 wt.%) was added, the entire sample immediately exhibited a uniform structural color, and it was found that a dispersion of opal-type colloidal crystals was formed.

### Example 8

To 200 µL of purified PNIPAM microgels (3.56 wt.%) prepared in the same manner as in Example 1, 5 to 30 mg each of a sodium polyacrylate (NaPAA, PAA average molecular weight of 1000000, neutralization degree of 50%) aqueous solution (prepared with an NaPAA concentration = 5 wt.%) was added the entire sample uniformly exhibited a structural color, and it was found that a dispersion of opal-type colloidal crystals was formed.

### Comparative Example 3 and Comparative Example 4

In contrast, to 250 µL of purified PNIPAM microgels (3.56 wt.%) prepared in the same manner as Example 1, 25 mg of an aqueous solution (5 wt.%) of NaPAA (neutralization degree of 50%) having an average molecular weight of 25000 in Comparative Example 3 and a molecular weight of 5000 in Comparative Example 4 was added a structural color was not observed, and it was found that a dispersion of opal-type colloidal crystals was not formed. As a cause for the failure to form the dispersion, it is thought that, due to the low molecular weight of the NaPAA molecules, the NaPAA molecules were able to penetrate into the space that was formed when the PNIPAM microgels were in close proximity to each other, and therefore a depletion region was not formed, failing to create a difference in osmotic pressure.

### Preparation of a Copolymerized Microgel Dispersion

NIPAM-AAm copolymer gel dispersions of Synthesis Examples 1 to 4 described below were synthesized as microgel dispersions.

### Synthesis Example 1

A NIPAM-AAm copolymer microgel dispersion was prepared by using, as monomers, 85 mol% of NIPAM, 10 mol% of acrylamide, and 5 mol% of BIS, and then adding SDS at a ratio of 1.5 wt.% and KPS at a ratio of 0.6 wt.%, with the other conditions being the same as in Example 1.

### Synthesis Example 2

A NIPAM-AAm copolymer microgel dispersion was prepared by using, as monomers, 75 mol% of NIPAM, 20 mol% of acrylamide, and 5 mol% of BIS, and then adding SDS at a ratio of 3.0 wt.% and KPS at a ratio of 0.6 wt.%, with the other conditions being the same as in Example 1.

### Synthesis Example 3

A NIPAM-AAm copolymer microgel dispersion was prepared by using, as monomers, 85 mol% of NIPAM, 10 mol% of acrylamide, and 5 mol% of BIS, and then adding SDS at a ratio of 1.0 wt.% and KPS at a ratio of 0.6 wt.%, with the other conditions being the same as in Example 1.

### Synthesis Example 4

A NIPAM-AAm copolymer microgel dispersion was prepared by using, as monomers, 75 mol% of NIPAM, 20 mol% of acrylamide, and 5 mol% of BIS, and then adding SDS at a ratio of 2.0 wt.% and KPS at a ratio of 0.6 wt.%, with the other conditions being the same as in Example 1.

The particle diameters of the NIPAM-AAm copolymer microgel dispersions of Synthesis Examples 1 to 4 prepared as described above were determined by measuring the diffusion coefficient by dynamic light scattering, and then entering the value thereof into the Stokes-Einstein equation. The results are shown in Table 1.

**[Table 1]**

| Synthesis Example | NIPAM | AAm | Bis | SDS | KPS | Particle Diameter |
|---|---|---|---|---|---|---|
| 1 | 85 mol% | 10 mol% | 5 mol% | 1.5 wt% | 0.6 wt% | 271 ± 27.3 nm |
| 2 | 75 mol% | 20 mol% | | 3.0 wt% | | 291 ± 53.7 nm |
| 3 | 85 mol% | 10 mol% | | 1.0 wt% | | 320 ± 36.1 nm |
| 4 | 75 mol% | 20 mol% | | 2.0 wt% | | 335 ± 21.1 nm |

### Dispersion Test in an Aqueous Solvent of Microgel and Measurement of the Reflection Spectrum

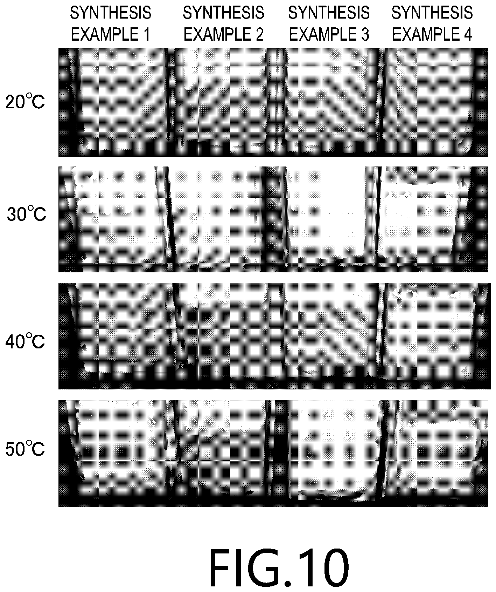

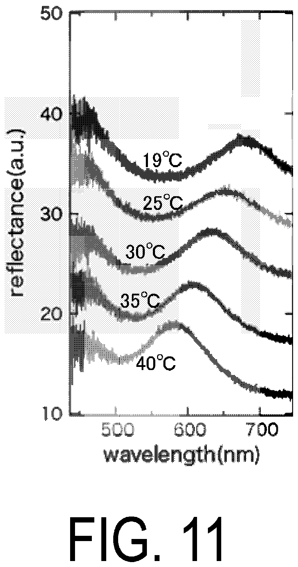

The microgel dispersions prepared in Synthesis Examples 1 to 4 were lyophilized into a powder, and the microgels were dispersed in ethanol aqueous solutions each having concentrations of 0, 10, 20, 30, 40 and 50 wt.% such that the concentration of the microgels became 2 wt.%. As a result, as shown in FIG. 10, with all of the dispersions, in a temperature range of from 20°C to 50°C, the microgels were dispersed without aggregating, and color development due to interference of the opal-type colloidal crystals was observed. Furthermore, when the reflection spectrum of the dispersion of Synthesis Example 4 was measured at different temperatures, as illustrated in FIG. 11, reflection peaks were observed up to at least 50°C, and the appearance maintained its color development as well.

### Example 9

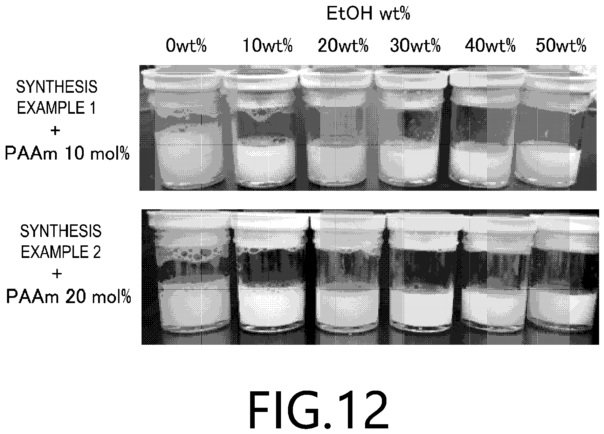

In Example 9, the microgel dispersions of Synthesis Example 1 and Synthesis Example 2 were lyophilized into powders and then dispersed in water to a concentration of 2 wt.% to form dispersions. Next, PAA was added at room temperature to these dispersions such that the PAA concentration was 10 mmol/L or 20 mmol/L. As a result, as shown in FIG. 12, interference colors associated with the formation of opal-type colloidal crystals were observed. Note that when the ethanol concentration was 30 wt.% or higher, the volume of the crystalline portion was reduced due to the shrinkage of the microgels, and a supernatant was observed. Thus, it was possible to maintain color development even when ethanol was added. In cases in which the ethanol concentration was 60 wt.% or higher, the PAAm did not dissolve. Regarding temperature resistance, microgels that could withstand 50°C were successfully synthesized. The sample ultimately exhibited white turbidity at 54°C. Regarding ethanol, the microgels were not able to withstand 50 wt.% ethanol, but tolerability was improved to 30 wt.%. Only samples of 30 wt.% ethanol exhibited dramatic precipitation. Microgels were dispersed in 60 wt.% to 100 wt.% ethanol, but the PAAm added as a polymer is a poor solvent with respect to ethanol and did not dissolve.

### Example 10

A decorative aqueous composition of Example 10 was prepared by the following operations.

First, the microgel dispersion prepared in Synthesis Example 3 was lyophilized into a powder, and then dispersed in ethanol aqueous solutions having concentrations of 0, 10, 20, 30, 40 and 50 wt.% such that the microgel concentration was 2 wt.%. Hydroxyethyl cellulose (molecular weight: 500000, product name: SE550, available from Daicel Corporation) was added to the dispersion at a concentration of 0.5 wt.%.

### Examples 11 to 13

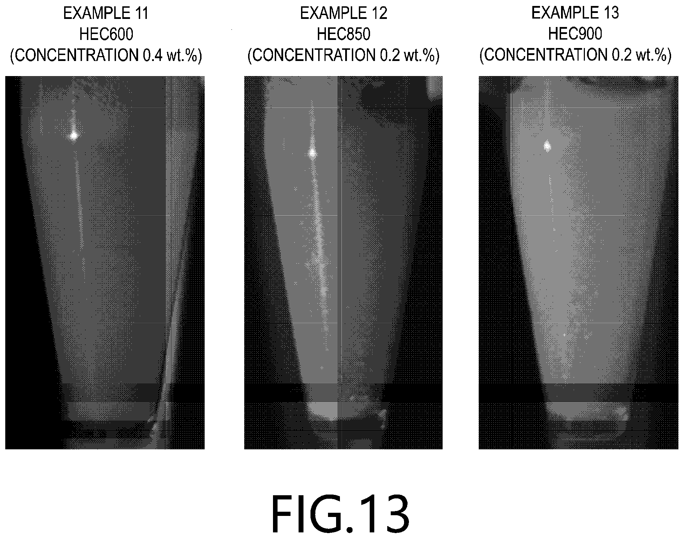

For the decorative aqueous composition of Example 11, hydroxyethyl cellulose having a different molecular weight than that of Example 10 was used (in Example 11, product of the trade name SE600, available from Daicel Corporation, having a molecular weight of 1020000 was used, in Example 12, product of the trade name SE850, available from Daicel Corporation, having a molecular weight of 1480000 was used, and in example 13, product of the trade name SE900, available from Daicel Corporation, having a molecular weight of 1560000 was used). Examples 11 to 13 were prepared with the other details being the same as those of Example 10. As a result, as shown in FIG. 13, interference colors associated with the formation of opal-type colloidal crystals were clearly observed.

### Example 14

To 500 µL of a purified PNIPAM gel (diameter of 193 nm, 3.56 wt.%) prepared in the same manner as in Example 1, 0.05 g of a 20 wt.% solution of polyethylene glycol (average molecular weight of 500000) was added, the entire sample exhibited a uniform structural color, and it was found that a dispersion of opal-type colloidal crystals can be prepared even when using polyethylene glycol as a water-soluble polymer.

### Relationship between the concentration of the polymer dissolved in the decorative aqueous composition and the lattice spacing

In order to examine the relationship between the concentration of the polymer dissolved in the decorative aqueous composition and the lattice spacing, the following experiment was conducted.

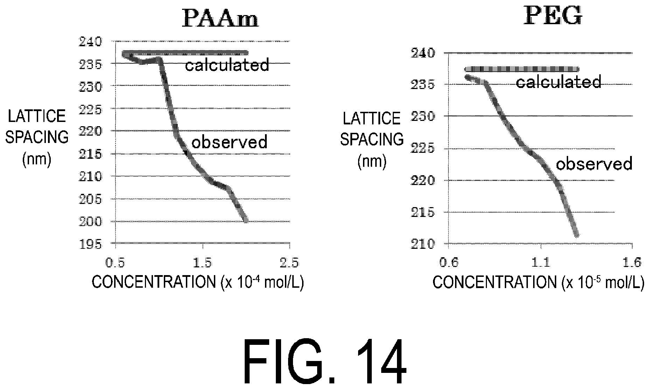

First, a water dispersion of the NIPAM microgels produced by the method of Example 1 was prepared, and PAAm having a molecular weight of 400000 (or PEG having a molecular weight of 1000000) was added thereto, and the lattice spacing was determined from a measurement of the Bragg diffraction wavelength. Meanwhile, the inter-particle distance for a case in which the size of the microgels was constant was determined through calculations. The results are shown in FIG. 14. From the graphs of FIG. 14, it was found that as the concentration of PAAm (or PEG) increased, the lattice spacing became smaller.

### Solidification of Dispersion of Opal-type Colloidal Crystals

The dispersions of the opal-type colloidal crystals of each of the examples described above can be immobilized by a known method (JP 2006-182833: Gel immobilized colloidal crystals). A specific example thereof is presented below.

The following chemical agents are added and dissolved in the dispersion of the opal-type colloidal crystals of each example.
Gel monomer: N,N'-dimethylol acrylamide (N-MAM)
0.67 mol/L
Crosslinking agent: methylenebisacrylamide (BIS) 10 mmol/L
Photopolymerization initiator: 2,2'-azobis [2-methyl-N-[2-hydroxyethyl]-propionamide
4 mg/mL
Sodium dodecyl sulfate: 10 µmol/L

Then, the gel monomer is polymerized by irradiating the dispersion of the above-described composition with ultraviolet light, and a solidified body of the dispersion of opal-type colloidal crystals is obtained.

The present invention is not limited in any way to the description of the embodiments and examples of the invention described above. Various modified aspects that are within a scope that could be readily conceived of by a person skilled in the art are also included in the present invention without departing from the scope of the claims.

### Industrial Applicability

The decorative aqueous composition according to the present invention contains opal-type colloidal crystals dispersed in an aqueous dispersion medium, and exhibits a glittering, aesthetically excellent structural color through Bragg diffraction. In addition, because the structural color thereof does not change at a salt concentration of about 0.1 mM, the decorative aqueous composition can be suitably used in cosmetic products such as cosmetic lotions.

### Reference Signs List

- 1: Aqueous dispersion medium
- 2: Polymer
- 3: Opal-type colloidal crystal
- 4,10: Colloidal particle
- 11: Polymer
- 12: Crosslinking agent
- 13: Crosslinking point
- S1: Microgel preparation
- S2: Dialysis
- S3: Colloidal crystallization

## Claims

1. A decorative aqueous composition comprising opal- type colloidal crystals dispersed in an aqueous dispersion medium, a polymer being dissolved in the aqueous dispersion, wherein colloidal particles constituting the opal-type colloidal colloidal crystals are dispersible in the aqueous dispersion medium and have an average particle diameter ranging from 10 nm to 1000 nm, and a variation coefficient of the particle diameter is within 20%.

2. The decorative aqueous composition according to claim 1, wherein a specific gravity of the colloidal particles is in a range from 0.9 times to 1.1 times a specific gravity of the aqueous dispersion medium.

3. The decorative aqueous composition according to claim 1 or 2, wherein the colloidal particles include a crosslinked polymer.

4. The decorative aqueous composition according to any one of claims 1 to 3, wherein the colloidal particles include poly(N-isopropylacrylamide).

5. The decorative aqueous composition according to any one of claims 1 to 3, wherein the colloidal particles include poly(N-isopropylacrylamide) and polyacrylamide.

6. The decorative aqueous composition according to any one of claims 1 to 5, wherein the aqueous dispersion medium includes an alcohol in addition to water.

7. A decorative solidified body formed by solidifying the decorative aqueous composition of any one of claims 1 to 6.

8. A method for producing a decorative aqueous composition, the method comprising mixing an aqueous dispersion medium, a polymer soluble in the aqueous dispersion medium, and colloidal particles having an average particle diameter ranging from 10 nm to 1000 nm with a variation coefficient of the particle diameter being within 20%, to form a dispersion of particles including opal- type colloidal colloidal crystals.

9. The method for producing a decorative aqueous composition according to claim 8, wherein the hydrophilic colloidal particles are a crosslinked hydrophilic polymer.
